# EUROPEAN PATENT APPLICATION

(11) **EP 1 582 213 A1**
(43) Date of publication of application: **05.10.2005**
(21) Application number: 04007827.1
(22) Date of filing: 31.03.2004
(51) Int. Cl.: A61K 31/7076

(54) **Use of adenosine for treating surgery related ischemia**

(71) Applicant: Ludwig-Maximilians-Universität München, 80539 München (DE)
(72) Inventor: Thiel, Manfred, 82223 Eichenau (DE); Chouker, Alexander, 81667 München (DE)
(74) Representative: Bohmann, Armin K.

(57) **Abstract**

The present invention is related to the use of adenosine for the manufacture of a medicament for the treatment of a human condition whereby the condition is selected from the group comprising surgery, ischemia, trauma, and hypoxia, characterised in that the adenosine is administered to the patient prior to the condition and after the condition.

## Description

The present invention is related to the use of adenosine for the manufacture of a medicament and methods for systemic administration of adenosine.

Adenosine has attracted considerable attention in the medical art as a local hormone with numerous tissue-specific biological functions. In myocardium, adenosine is released in small amounts at constant basal rate during normoxia. During ischemia the production of adenosine increases several fold due to breakdown of adenosine triphosphate (ATP). Increased production of adenosine causes coronary vasodilatation. Thus, adenosine couples myocardial metabolism and flow during ischemia and is called a homeostatic or "retaliatory metabolite". Furthermore, adenosine has electrophysiological effects in supraventricular tissue, causing a decrease in heart rate. In 1985, it was discovered that adenosine also exerts cardioprotective effects directly on cardiomyocytes.

The involvement of adenosine has led to a number of various uses of adenosine in the medical field. Among others, adenosine is used to treat cardiac dysrhythmia and accelerated heart rate. Also, adenosine preconditions human myocardium against ischemia in vivo as may happen, for example, in percutaneous transluminal coronary angioplasty (PTCA) and is used prophylactically to attenuate ischemia in patients undergoing PTCA of the left anterior descending coronary artery (Leesar M. A., et al., Circ. 1997 Jun 3; 95 (11): 2500-7). The pretreatment of adenosine induces an early ischemic preconditioning effect against ischemia.

The problem underlying the present invention was thus to find further medical indications and conditions in the treatment and/or prevention of which adenosine can be further used.

According to the present invention this problem is solved in a first aspect by the use of adenosine for the manufacture of a medicament for the treatment of a human condition whereby the condition is selected from the group comprising surgery, ischemia, trauma, and hypoxia, characterised in that the adenosine is administered to the patient prior to the condition and/or after the condition.

In an embodiment the adenosine is systemically administered.

In an preferred embodiment the systemic administration is performed through intravenous administration.

In an embodiment the adenosine is present in a liquid solution.

In an embodiment the condition is surgery.

In an preferred embodiment where the condition is surgery, the adenosine is administered from about 30 to 20 minutes until about 10 to 5 minutes prior to the surgery.

In a further preferred embodiment where the condition is surgery, the adenosine is administered starting from immediately after the surgery until about 60 minutes after the surgery, whereby the administration lasts for a period of time of from about 30 minutes to about 360 minutes.

In an embodiment the surgery is selected from the group comprising liver surgery, heart surgery, lung surgery, kidney surgery, skin surgery, intestinal surgery, and pancreas surgery.

In an embodiment the condition is surgery and involves a second condition.

In a preferred embodiment the second condition is selected from the group comprising ischemia, reperfusion damage, hypoxia, and shock.

In an embodiment the surgery is transplantation.

In a preferred embodiment where the surgery is transplantation, the donor organ is prior and/or during the removal from the donor organism treated with adenosine, preferably for a period of time from about 30 to 20 minutes to about 10 to 5 minutes prior to the removal of the donor organ.

In an embodiment the condition is trauma.

In a preferred embodiment the trauma results from the handling of an organ or part thereof.

In an embodiment the condition is ischemia.

In a preferred embodiment where the condition is ischemia, the adenosine is administered for about 10 minutes to the patient about 20 to 10 minutes prior to ischemia.

In a further preferred embodiment where the condition is ischemia, the adenosine is administered to the patient for about 70 to 90 minutes after ischemia beginning about 1 to 5 minutes after removal of the ischemia causing blockade.

In an embodiment the condition is hypoxia.

In an embodiment where the condition is hypoxia, ischemia or trauma, the condition is arising from a surgery.

In a preferred embodiment the condition is part of a medical treatment scheme, preferably involving surgery.

In a preferred embodiment the condition is an accidental event during such medical treatment scheme.

In an embodiment the condition is an intentional event during such medical treatment scheme.

In a preferred embodiment the medical treatment scheme is related to the prevention and/or treatment of cerebral ischemia, trauma, hypoxia, acute Cor pulmonale, chronic Cor pulmonale intracerebral aneurysms.

In an embodiment the condition is part of a medical treatment scheme in which reanimation or the use of a heart-lung machine is involved.

According to the present invention this problem is solved in a second aspect by the use of adenosine for the manufacture of a medicament for the treatment of multi-organ failure.

In an embodiment of the second aspect the multi-organ failure is an accidental or an intentional event during a medical treatment scheme.

In an embodiment of the second aspect the adenosine is systemically administered.

According to the present invention this problem is solved in a third aspect by a method for systemic administration of adenosine to a patient comprising the steps of
(a) determining the hemodynamic situation of the patient;
(b) administering to the patient an amount of adenosine;
(c) determining the hemodynamic situation of the patient;
(d) comparing the hemodynamic situation of the patient with an undesired hemodynamic situation;
(e) repeating steps (b) to (d) in case the hemodynamic situation of the patient is different from the undesired hemodynamic situation, whereby the amount of adenosine administered is higher than the amount of adenosine administered in step (b)

In an embodiment of the third aspect the undesired hemodynamic situation is selected from the group comprising bradycardia, arrhythmia, and arterial hypotension.

In an embodiment of the third aspect, upon realization or detection of the undesired situation for a period of time of at least 30 seconds, the amount of adenosine is administered which led to the undesired hemodynamic situation.

In an embodiment of the third aspect the adenosine is continuously administered.

In a preferred embodiment of the third aspect, upon another occurrence of an or of the undesired hemodynamic situation, the amount of adenosine administered is adjusted to the one which does not trigger the undesired hemodynamic situation.

In a further preferred embodiment of the third aspect, after about at least 30 seconds upon adjusting the amount of adenosine which does not trigger the undesired hemodynamic situation, the amount of adenosine administered is increased to the amount which triggers the undesired hemodynamic situation.

According to the present invention this problem is solved in a fourth aspect by the use of the method according to the third aspect of the present invention for the administration of adenosine as described in any of the first and the second aspect.

The present inventors have surprisingly found that adenosine, particularly if administered systemically, can be used in the treatment of a number of various medical indications, i. e. medical conditions observed in the human being beyond those described in the prior art. This finding is, among others, and without wishing to be bound by any theory, based on the insight gained by the present inventors that externally administered adenosine is a pleiotropically acting compound which realizes the concept of a multi-drug-regimen in itself, particularly with regard to cytoprotective effects under various human conditions. Insofar the present inventors clearly deviate from the path outlined in the art where the use of adenosine was deemed to be limited to influencing myocardial tissue and the treatment of ischemia and ischemic preconditioning. Also, the present inventors have perceived that the prejudice in the art that the use of adenosine may result in arterial hypotension and/or decrease of the heart rate, can be controlled by a systemic administration of the adenosine, more particularly according to the regimen disclosed herein, and thus increase the safety of using adenosine which has been questioned in the past by those skilled in the art and thus avoided a broader application of adenosine in the treatment of human conditions.

Any accidental event during a medical treatment scheme can, for example, preferably be any complication which results in an undesired or unintended damage of the tissue, preferably through trauma, ischemia and hypoxia. Such complication may arise, e. g., from accidental cutting of vessels, or occlusion of vessels or arrest of haemorrhage.

As used herein the term human condition means a condition of a human being, preferably a condition experienced by human being including any individual parts thereof such as an organ or a tissue thereof. In a first sub-aspect of the present invention such human condition is surgery, i. e. an injury of the human body for restoring the physical or psychological health.

It is within the present invention that such surgery is particularly a surgery selected from the group comprising liver surgery, heart surgery, lung surgery, kidney surgery, skin surgery, intestinal surgery, and pancreas surgery, however, not limited thereto. In a particularly preferred embodiment, the surgery is transplantation. More particularly, surgery in transplantation is to be understood as surgery performed on the donor organism and includes also, separate and independent therefrom, surgery performed on the recipient organism.

It is also within the present invention that surgery is any emergency surgery as well as an elected surgery. As used herein, emergency surgery is in an embodiment of a surgery which is to be performed due to a medical emergency and is thus particularly not a surgery or procedure the time at which it has to be performed is planned in advance, but is necessary due to an unexpected event making such procedure necessary preferably to respond to a serious risk for the health of the human being or patient. In contrast to this, an elect surgery is a surgery which is, preferably, necessary from the medical point of view, however, the timing of which can be determined by the medical doctors and is not triggered by the existence of a situation which requires immediate or rather close action so as to avoid any deleterious effects to the human or patient body.

It is within the present invention that the particular human condition according to the first sub-aspect of the present invention, i. e., surgery, can be accompanied by a second condition. Such second condition is either one selected from the group comprising ischemia, reperfusion damage, hypoxia and shock or any combination thereof.

Said second condition can occur during the first condition, e. g. during surgery or any other human condition or any other first condition defined herein, either intentionally or unintentionally, such as by accident. In a preferred embodiment, an unintentional second condition is a condition which happens during the first condition, i. e. the surgery or any other human condition, due to the reaction of the body subject to said first condition or due to the measures applied to such body, without that the second condition is anticipated or intended by the medical practitioners performing the or being responsible for the first condition. Any accidental event during a medical treatment scheme can, for example, preferably be any complication which results in an undesired or unintended damage of the tissue, preferably through trauma, ischemia and hypoxia. Such complication may arise, e. g., from accidental cutting of vessels, or occlusion of vessels or arrest of haemorrhage.

In contrast to this, an intentional second condition is a condition the existence of which is anticipated or even caused by the medical practitioners performing the or being responsible for the first condition of the respective body. It may thus be necessary during a human condition such as a surgery to create an ischemia, for example, if vessels have to be occluded because, for example, an organ naturally connected to such vessel has to be replaced by a substitute organ.

The same is true also for reperfusion damage which can arise from the intended occlusion of a blood vessel, or for hypoxia which, despite further measures such as hibernation are realized, can occur to a tissue, sometimes even intentionally. Insofar an intentional event during such medical treatment scheme can be any intended tissue damage through trauma or ischemia which is part of the procedure performed according to the medical art.

It is within the present invention that these second conditions occur or may occur in connection with any first condition which is any of the human condition described herein, in each and any of its various embodiments.

According to the second sub-aspect of the present invention the human condition is trauma. In a preferred embodiment trauma as used herein, is any injury which happens to a human or animal body, preferably a human body. In a preferred embodiment, trauma is different from what a human body is experiencing upon PTCA. It is also within the present invention that trauma comprises an injury performed on a body undergoing surgery. In other words, surgery can be a part of a condition which is referred to herein as trauma. It is also within the present invention that trauma being a first condition. As for each and any of the human conditions disclosed herein, also trauma as a first condition can, in a preferred embodiment, also comprise a second condition. The second condition can be any such second condition as described in connection with the first sub-aspect of the present invention, where the condition is surgery. In a particularly preferred embodiment, the trauma results from the handling of an organ or parts thereof, either in connection with transplantation or in connection with surgery, however, in both cases not limited thereto. It is to be acknowledged that the handling of organs in trauma or surgery causes severe changes to the tissue and organ, respectively thus handled. This is particularly true for the inner organs such as heart, lung, kidneys, liver, and the intestine and stomach. This kind of trauma can particularly occur during transplantation, but also during other forms of curative medical procedures such as surgery, including plastic surgery.

In a third sub-aspect the human condition is ischemia. As used herein in an embodiment, ischemia means the interruption of the blood flow to an organ, part of an organ or tissue, preferably because of an inappropriate arterial blood flow. Such inappropriate arterial blood flow may be caused by thrombosis, embolism, endarteritis obliterans, vessel spasm, tumors and the like. In an other embodiment, ischemia also comprises reperfusion and the condition arising from such reperfusion.

It is also within the present invention that ischemia is accompanied by a second condition, whereby the second condition is in principle one of those discussed in connection with the first sub-aspect of the present invention, i. e. where the first condition is surgery. Also, both the first condition, i. e. ischemia, as well as the second condition can, independently from each other, be intentional or unintentional as defined herein.

In a fourth sub-aspect, the human condition is hypoxia which, also, can be accompanied by a second condition corresponding to the second conditions discussed in connection with the first sub-aspect of the present invention, where the first condition is surgery. Again, the first and second condition can, independently from each other, be intentional or unintentional as defined herein.

It is also within the present invention that in an embodiment the human condition of the second sub-aspect, i. e. trauma, the human condition of the third sub-aspect, i. e. ischemia, and the condition of the fourth sub-aspect, i. e. hypoxia, are linked to or arise from the human condition of the first sub-aspect, i. e. surgery, as defined herein.

It is also to be acknowledged that any of the aforementioned first conditions and any of the second conditions disclosed herein, either alone or in any combination, can be part of a medical treatment scheme and arise either as an accidental or unintentional event during such medical treatment scheme or as an intentional event during such medical treatment scheme.

In a preferred embodiment the medical treatment scheme is a treatment of any human condition, preferably a human condition as defined herein, or a measure suitable to prevent any human condition, preferably a human condition, a first condition or a second condition as defined herein. Although factually any disease can involve a surgery, particularly preferred medical treatment schemes where any of these conditions may happen, are cerebral ischemia, trauma, hypoxia, acute Cor pulmonale, chronic Cor pulmonale and intracerebral aneurysms. Also, said first and second conditions, either alone or in combination, can arise from a medical treatment scheme in which reanimation or the use of a heart-lung machine is involved.

In a further aspect, the present invention is related to the use of adenosine for the manufacture of a medicament for the treatment of multi-organ failure. Such multi-organ failure can happen prior to, during or after any of the first and second conditions disclosed herein. It is within the present invention that such multi-organ failure is an accidental or an intentional event during a medical treatment scheme. For instance multi-organ failure may develop accidentally as a consequence of successful resuscitation after cardiac arrest, hemorrhagic-traumatic shock, or sepsis. Intentional events that are associated with the development of multi-organ failure comprise local or systemic ischemia-reperfusion following aortal aneurysectomy or usage of extracorporal circulatory assist devices.

In a still further aspect the present invention is related to the use of adenosine in sports medicine and aviation medicine. In a preferred embodiment, the use of adenosine in the field of sports medicine increases the stamina of a person and, particularly with regard to the overall tissue protective effects of the adenosine, allows the person to recover faster from a physical stress condition going along with physical exercises during sports. It is also within the present invention to use adenosine as described in any aspect herein prior, during and/or after physical exercise at high altitude or after diving such as for the treatment of caisson illness, or even after near-drowing, which are all situations that are exacerbated by ischemically or hypoxically triggered tissue inflammation. The same applies also to the use of adenosine in aviation medicine. Treatment of humans with adenosine as disclosed herein in connection with any other aspect of the present invention, might help to render them less susceptible to a subsequent period of hypoxia under planned or emergency conditions that may be experienced by pilots in combat jets or passengers during space flight, preferably to space stations in the orbit or beyond.

The adenosine which is used according to the present invention is typically a synthetic adenosine, preferably an adenosine which has been synthesized by chemical procedures. However, it is also within the present invention that the adenosine may be a natural adenosine, more preferably an adenosine which has been taken from a biological source. Such a kind of natural adenosine may be generated upon degradation of its phosphorylated forms such as from AMP or ADP, which can also be infused into the patient. It is also within the present invention that derivatives of adenosine are used as disclosed herein for adenosine, provided that the respective derivate of adenosine has the same pharmacologic properties which are responsible for the effects observed herein, in particular the multi-drug regimen. Adenosine derivatives as such are known to the ones skilled in the art and include, but are not limited to ATP, ADP and AMP.

In a preferred embodiment, the adenosine is administered systematically to a living being, preferably an animal or a human being, even more preferably a human organism. Such administration occurs preferably through an intravenous administration. The administration of adenosine is typically performed by means of intravenous administration, either via a peripheral vein or more preferably through a central venous line

Adenosine is preferably administered in a liquid solution. More preferably, such liquid solution contains 2% v/w and is typically contained in a buffer solution. However, the concentration range may vary between 0,02% to 5 % according to the infusion needs. It is also within the present invention that the adenosine may form part of infusion solutions which are known in the art. Preferably, such infusion solutions contain also salts, nutrients, vitamins and/or a pharmaceutically active compound. Preferably, the pharmaceutically active compound is selected from the group comprising anti-inflammatory drugs such as antioxidants, xanthin oxidase inhibitors, special fatty acids, immunosuppressive cytokines, cAMP raising agents, and synthetic sympathomimetics.

The administration of adenosine goes along with an analgetic effect of adenosine so that apart from the uses described herein, adenosine may also be used as a subsituent to analgetic compound, including, but not limited to, codeine and codeine derivatives such as dihydrocodeine and oxycodone.

It is also within the present invention that the adenosine is administered using other sedatives or anaesthetics or anaesthetic gases.

In connection with the use of adenosine as envisaged by the present inventors, it is to be acknowledged that the lifetime of adenosine is rather short so that long lasting effects are avoided. Additionally, the short lifetime of adenosine is also insofar advantageous as the effect of adenosine can be antagonized immediately by administering, e. g., theophylline.

It is to be acknowledged that the adenosine is administered in various embodiments according to different schemes. It is also within the present invention that any of the schemes described for one human condition where adenosine is administered, can in principle also be used for any of the other human conditions and first and second conditions, respectively, as described herein. In case of the condition being surgery, the adenosine is preferably administered from about 30 to 20 minutes prior to the surgery and stopped about 5 to 10 minutes prior to the surgery. In an embodiment of the present invention, no adenosine is administered after the surgery. In an alternative embodiment the adenosine is administered after the surgery. More preferably, the adenosine administration is started at any time after the surgery to about 60 minutes after the surgery, whereby, preferably, the administration of the adenosine lasts from about 30 minutes to about 360 minutes.

In an other embodiment, the adenosine is administered during the surgery irrespective of any other adenosine administration prior or after the surgery.

It is also within the present invention that the adenosine is administered prior, during and after the surgery, that the adenosine is administered prior and during the surgery, during and after the surgery and during and after the surgery, with the time periods for adenosine administration being as described herein for the individual adenosine administration steps or phases. The same scheme is applicable in preferred embodiments also to any other human condition, including any first and second condition as described herein.

In case of ischemia, the adenosine is preferably administered for about 10 minutes to the patient about 10 to 20 minutes prior to ischemia.

In case of transplantation, a particularly preferred administration scheme for adenosine is such that the donor organ is treated with adenosine as described herein prior and/or during the removal from the donor organism. Also, the recipient organism is treated using adenosine according to the technical teaching disclosed herein, preferably the one described for surgery.

It is to be acknowledged that the term after surgery refers to a point in time, where a second condition has intentionally or unintentionally been terminated no longer ago than 1 to 360 minutes.

It is also to be acknowledged that the administration of adenosine as disclosed herein can be performed, for each condition, preferably for each human condition, first condition and second condition as described herein, according to the present invention prior to the condition to be treated and/or after such condition. Additionally and independent therefrom the administration can occur additionally to any of the adenosine administration scheme also performed during such condition.

The present inventors have furthermore discovered, that the cytoprotective effects described herein which result in the particular uses disclosed herein, can be particularly effective if adenosine is administered according to a certain administration regimen. Therefore, it is within the present invention that in the various uses according to the present invention the adenosine is administered according to the method and regimen according to the present invention, and the method and regimen according to the present invention is used to administer the adenosine for the various uses according to the present invention. A preferred embodiment of such regimen uses intravenous administration of adenosine through a port, which is preferably located close to the heart, e.g. via a central venous line, where a solution of 0.2% w/v of adenosine is administered. Preferably such administration is performed through an infusion apparatus so as to provide a controlled administration. A more preferred administration scheme for adenosine reads as follows:
30 seconds: 30 µg adenosine/kg body weight/min,
30 seconds: 60 µg adenosine /kg body weight/min,
30 seconds: 90 µg adenosine /kg body weight/min,
60 seconds: 120 µg adenosine /kg body weight/min,
5 minutes: 150 µg adenosine /kg body weight/min,
3 minutes: 120 µg adenosine /kg body weight/min, and
subsequently 150 µg adenosine /kg body weight/min.

It is within the present invention that based on this scheme or regimen also different schemes can be developed, whereby the overall amount of adenosine is to be taken into consideration, particularly the overall amount of adenosine administered within the periods specified resulting in regimen that can induce equieffective desired effects

In a further aspect the present invention is related to a method for systemic administration of adenosine to a patient. Such method comprises the following steps:
(f) determining the hemodynamic situation of the patient;
(g) administering to the patient an amount of adenosine;
(h) determining the hemodynamic situation of the patient;
(i) comparing the hemodynamic situation of the patient with an undesired hemodynamic situation;
(j) repeating steps (b) to (d) in case the hemodynamic situation of the patient is different from the undesired hemodynamic situation, whereby the amount of adenosine administered is higher than the amount of adenosine administered in step (b).

It is within the present invention that the undesired hemodynamic situation is selected from the group comprising brachycardia, arrhythmia, and arterial hypotension. As used herein, brachycardia means in a embodiment a heart rate of less than 45/minutes. As used herein, arrhythmia is preferably defined as an atrioventricular block higher than first grade, and arterial hypotension preferably means an arterial blood pressure below 50 mm Hg.

It is to be noted that upon realization or detection of the undesired situation adenosine is subsequently administered in an amount corresponding to the last amount administered not triggering the undesired hemodynamic situation for a period of time of at least 30 seconds, whereupon after that period of time the amount of adenosine is administered which led to the undesired hemodynamic situation. This approach is based on the further surprising finding of the present inventors that due to this modulation, typically the organism is conditioned so as to accept a higher titer of adenosine being an effective cytoprotective agent still avoiding the undesired hemodynamic situation as described above.

It is to be acknowledged that this procedure is repeated whenever such undesired hemodynamic situation occurs again. If, however, in a preferred embodiment the undesired hemodynamic situation arises at least two times in a respective cycle, this is an indication that the amount of adenosine which may be administered to the particular organism, has reached its limit. Concomitantly the amount of adenosine which is subsequently administered to the organism does not trigger the undesired hemodynamic situation, preferably the one which is the highest one not triggering said undesired hemodynamic situation. In a preferred embodiment, the increase in the amount of adenosine administered to a patient is preferably within 5 to 90 µg/kg body weight/min. The increment typically chosen for increasing the amount of adenosine administered preferably is around 30 µg/kg body weight/min adenosine.

It is to be understood that any aspect of the present invention is applicable to both the animal and human body, and both to a healthy as well as diseased organism, whereby the human organism is preferred. A diseased organism is also referred to herein in a preferred embodiment as patient.

The present invention is further illustrated by the examples and figures from which further features, embodiments and advantages of the present invention may be taken, whereby
- Fig. 1: shows a graph depicting two adenosine infusion regimens shown as step-wise adenosine application as a function of time;
- Fig. 2: shows a diagram depicting the interleukin 6 titre in the blood of the four patient groups pre liver resection and on the first and second day after the resection; and
- Fig. 3: shows a diagram depicting the expression of beta 2 adhesion molecules on circulating granulocytes depicted as relative units on granulocytes taken from the adenosine group, control group 1 and control group 2 prior to ischemia and 3, 30 and 120 minutes after reperfusion.

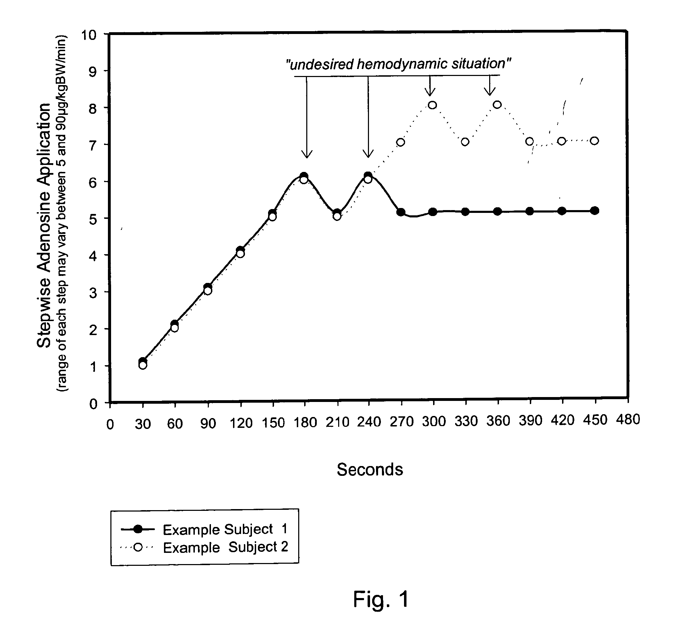

Fig. 1 shows a graph depicting two adenosine infusion regimens shown as step-wise adenosine application as a function of time. More particularly, over a range of about 450 seconds the infusion rate for two patients, referred to as example subject 1 and example subject 2, is depicted. The infusion rate of adenosine which was used as a 0.2 % aqueous adenosine solution, results in both cases from multiplying the number of steps practised at the particular time with the step-wise defined infusion rate which was between 5 and 90 µg/kg body weight/minute. In case of example subject 1 the infusion rate was increased in a step-wise manner for a total period of 180 seconds, whereby an increase was realized every 30 seconds. After 180 seconds, an undesired hemodynamic situation arose which was remedied by applying the highest infusion rate which did not cause the undesired hemodynamic situation. A further increase of the adenosine infusion rate to a level similar to the one where the undesired hemodynamic situation was observed for the very first time, resulted again in the undesired hemodynamic situation so that the further infusion rate kept for the next minutes was the one preceding immediately this latter one, i. e. the one where no undesired hemodynamic situation arose as realized at 210 seconds.

For example subject 2, the same strategy was realized, whereby once an undesired hemodynamic situation was reached at, in this case, 180 seconds, the infusion rate was decreased to an infusion rate where no such undesired hemodynamic situation arose and subsequently increased again to the rate where, when realized for the first time, the undesired hemodynamic situation had arisen, whereupon, this time, no such undesired hemodynamic situation arose. The maximum infusion rate was reached after 300 second where, again, an undesired hemodynamic situation arose and, the infusion rate was decreased to the last infusion rate where no such undesired hemodynamic occurred followed by the infusion rate which happened to cause the undesired hemodynamic situation at 300 seconds, whereupon subsequently the highest infusion rate was realized where no such undesired hemodynamic situation arose, namely the one realized at 270 and 330 seconds, respectively.

This is an example for an administration scheme for adenosine according to the present invention.

### Example 1: Cytoprotective effects of adenosine in partial liver resection

This example is related to the use of adenosine in partial liver resection and is according to the present inventors' best knowledge the first description where native adenosine is administered to a patient through intravenous perioperative infusion in such procedure.

The study conditions were allowed by the ethics commission and a written consent of every patient participating in the study has been provided after intense education.

### Study groups

Adenosine group: The members of this group were experiencing liver trauma during resection and additional organ ischemia during resection (Pringle-manoeuvre, duration 30 to 45 minutes). Liver protection was realised by intermitting central venous administration of adenosine (infusion duration 10 minutes, 120 to 150 µg/kg body weight/min) 20 minutes prior to resection/ischemia, and a new continuous infusion immediately after reperfusion (infusion duration 70 to 90 minutes, 120 to 150 µg/kg body weight/min).

Control group 1: The members of this group experienced a liver trauma during resection and additional organ ischemia during resection (Pringle- manoeuvre, duration 30 to 45 minutes). Liver protection was realised through ischemic pre-conditioning by occlusion of the liver supplying vessels (a single ten minutes lasting ischemia and 10 minutes reperfusion) starting 20 minutes prior to resection.

Control group 2: The members of this group experienced a liver trauma through resection and additional organ ischemia during resection (Pringle- manoeuvre, duration 30 to 45 duration). No measures for organ protection through administration of adenosine or ischemic pre-conditioning were taken.

Control group 3: The members of this group experienced a liver trauma through resection without any organ ischemia (no Pringle- manoeuvre) and no other measures for organ protection.

### Tests

The following tests were carried out in order to assess the post-operational organ functions:
1. Quick test
2. Determination of the bilirubin
3. Lactate for monitoring the energetic situation

Any of the results obtained by applying these tests on blood samples taken from the members of the various study groups, are depicted in table 1:

**Table 1:**

| Pre and postoperative ("1" first and "2" second postoperative day) Prothrombin Time (PT expressed as Quick-values), bilirubin (bili) und lactate-concentration, resection surface (Res-surface), duration of Pringle (T-prin). | | | | | | | | | |
|---|---|---|---|---|---|---|---|---|---|
| | Nor m | **Adenosine** | *SEM* | **Control 1** | *SEM* | **Control 2** | *SEM* | **Control 3** | *SEM* |
| PT(Quick)pr e | 70-100 % | 100 | *0* | 95 | *3,1* | 97 | *3,3* | 98 | *1,7* |
| PT(Quick)1 | | #*66 | *4,9* | *56 | *2* | *52 | *4,2* | *55 | *5* |
| PT(Quick)2 | | 72 | *7,8* | *67 | *3,2* | *57 | *9,2* | *62 | *7* |
| Lactat pre | <2 mmo I/I | <1,0 | | <1,0 | | <1,0 | | 1 | |
| Lactate-1 | | 1,8 | *0,5* | 1,3 | *0,2* | 3,1 | *1,7* | 1,6 | *0,7* |
| Lactate-2 | | 1,2 | *0* | 1,2 | *0,3* | 2,3 | *1,2* | 1,6 | *0,2* |
| bili pre | <1,0 mg/dI | 0,4 | *0,1* | 0,4 | *0,07* | 0,5 | *0,19* | 0,5 | *0,18* |
| bili-1 | | #0,6 | *0.12* | 0,1 | *0,35* | 1,7 | *0,24* | #0,7 | *0,26* |
| bili-2 | | #0,5 | *0,08* | 1,1 | *0,51* | 1,9 | *0,59* | 0,8 | *0,38* |
| | | | | | | | | | |
| Res-surface | | 104 | *48,4* | 102 | *11* | 128 | *39* | 121 | *1,7* |
| T-prin | | 35 | *5,5* | 37 | *2* | 37 | *3,2* | 34 | *9,8* |
| (Mean, SEM; paired T-Test, * p<0,05 vs. pre, # p<0,05 vs. Control 3), *Res-surfacse:* resection surface of the liver after liver was resected. | | | | | | | | | |

The quick test serves as a parameter for the assessment of the protein synthesis performance of the liver after the procedure. As may be taken from table 1, the value of the quick test on the first and second day after surgery is higher in the adenosine group corresponding to a better liver synthesis performance, than in any of the control groups. The effect arising from continuous infusion of adenosine was superior to the effect seen in control group 1 where the protective effect was generated through ischemic pre-conditioning. The improvement of the post-procedure protein synthesis performance of the liver, particularly on the second day after the surgery, is highest in the adenosine group compared to each and any of the other groups and is not significantly different from the pre-procedure values.

The limited secretion of bilirubin as may be taken from the amount of total bilirubin, is most pronounced after trauma, ischemia and reperfusion of the liver as experienced by control group 2 and is, in terms of statistics, significantly higher than the pre-procedure values. The increase in bilirubin, compared to control group 2, was significantly less pronounced when the liver was protected against trauma and ischemia and reperfusion damage through either ischemic preconditioning (control group 1) or when there was surgical liver trauma only without ischemia and reperfusion of the liver (control group 3). Surprisingly, the bilirubin concentration in the adenosine group remained unchanged compared to pre- procedure values and is within the physiologically tolerable ranges at any time and is also lower on the second and first post-procedure day compared to any of the control groups (p = 0.02 and p = 0.04 vs. control group 3).

Also using lactate as a parameter for cytoprotective effects confirms the significant effectiveness of adenosine administration. The lactate concentration in serum is regarded as a marker for the extent of anaerobic metabolism due to lack of oxygen caused by a cellular lack of energy. The lactate concentrations in the serum are highest post-procedure in group 2, where surgical liver trauma and ischemia/reperfusion was combined. The initial increase in the lactate on the first post-procedure day is less pronounced if the liver is protected, prior to resection and ischemia, through ischemic pre-conditioning or through intravenous systemic administration of adenosine prior and after resection and ischemia.

### Adenosine mediated anti-inflammation protection

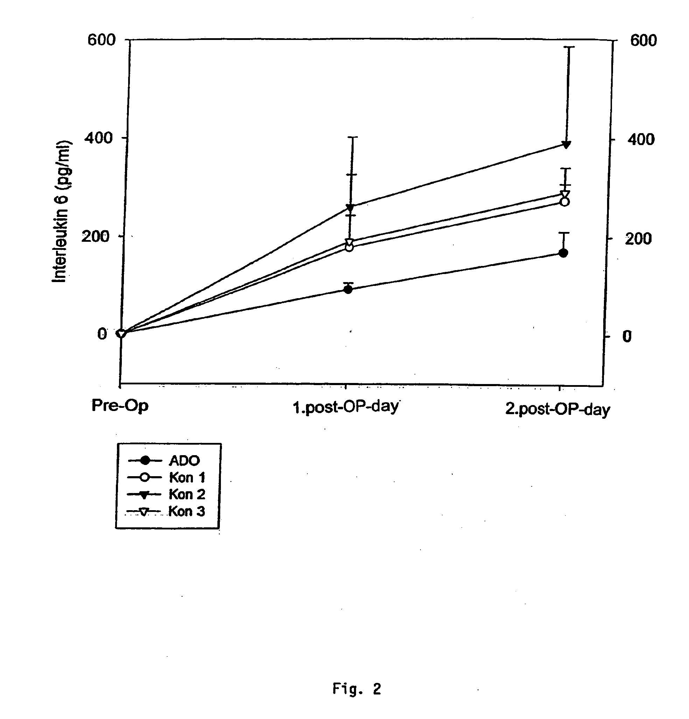

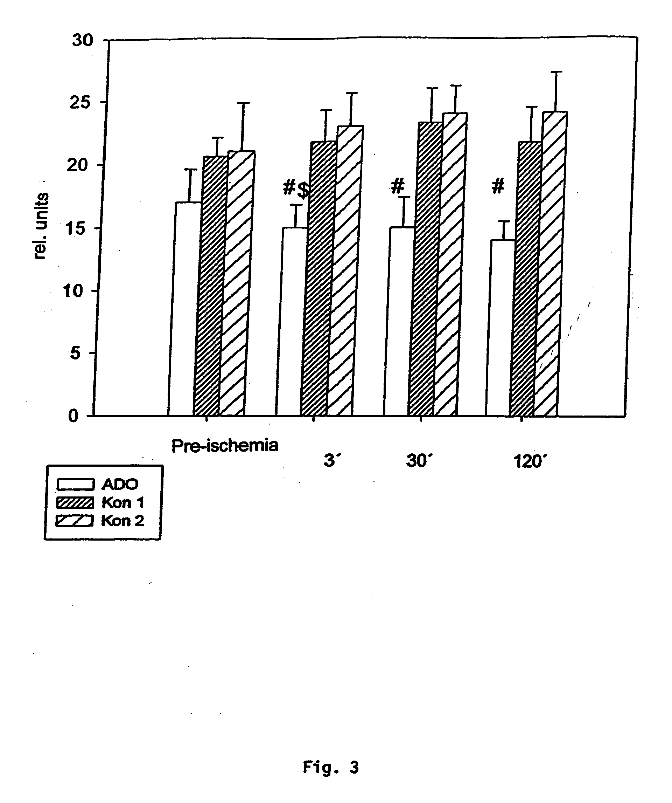

To assess protection conferred by adenosine on the anti-inflammation branch, both interleukin 6 and granulocyte functions were determined, whereby the results are presented in Figs. 2 and 3.

As depicted in Fig. 2, the level of interleukin 6 increased in any of the four groups, whereby each group consisted of three to five patients. Such increase is typically observed as a post-procedure reaction. The serum titres of the pro-inflammatory cytokine interleukin 6 can increase by three magnitudes compared to the pre-procedure titre and were highest in control group 2, where both surgical liver trauma and ischemia/reperfusion were combined. Protection of the liver through ischemic preconditioning as performed on control group 1, results in a less pronounced increase of the interleukin 6 level similar to the one observed for control group 3 where liver resection was performed without ischemia. However, the IL 6 titre was significantly less pronounced in the adenosine group, where prior and subsequent to resection adenosine was administered.

The further parameter for anti-inflammation, i. e. granulocyte function, was measured as the inhibition of potential cytotoxic functions of white blood cells due to liver ischemia which was measured using the expression of beta 2-intergrins through the monoclonal anti-CD18 antibody. More particularly, the expression was determined prior to ischemia and 3, 30 and 120 minutes after reperfusion. The cells were analysed using a FACScan and a FITC marked anti-CD18 antibody. The analysis as depicted in Fig. 3 shows the mean values including standard error, using T-test. As indicated in Fig. 3 $ p < 0.05 vs. control group 1, # p < 0.05 vs. control group 2.

The activation of granulocytes circulating in the blood which was observed immediately after ischemia and reperfusion (control groups 1 and 2) is effectively avoided through pre- and post-ischemic administration of adenosine, i. e. administration of adenosine prior and after ischemia. It is to be acknowledged that also the activation was somewhat less pronounced after ischemic pre-conditioning as realised in control group 1 (120 minutes).

The features of the present invention disclosed in the specification, the claims and/or the drawings may both separately and in any combination thereof be material for realizing the invention in various forms thereof.

## Claims

1. Use of adenosine for the manufacture of a medicament for the treatment of a human condition whereby the condition is selected from the group comprising surgery, ischemia, trauma, and hypoxia, **characterised in that** the adenosine is administered to the patient prior to the condition and after the condition.

2. Use according to any of claim 1, whereby the condition is surgery.

3. Use according to claim 2, whereby the adenosine is administered from about 30 to 20 minutes until about 10 to 5 minutes prior to the surgery.

4. Use according to claims 2 and 3, whereby the adenosine is administered starting from immediately after the surgery until about 60 minutes after the surgery, whereby the administration lasts for a period of time of from about 30 minutes to about 360 minutes.

5. Use according to any of claims 2 to 4, whereby the surgery is selected from the group comprising liver surgery, heart surgery, lung surgery, kidney surgery, skin surgery, intestinal surgery, and pancreas surgery.

6. Use according to any of claims 2 to 5, whereby the condition is surgery and involves a second condition.

7. Use according to claim 6, whereby the second condition is selected from the group comprising ischemia, reperfusion damage, hypoxia, and shock.

8. Use according to any of claims 2 to 7, whereby the surgery is transplantation.

9. Use according to claim 8, whereby the donor organ is prior and/or during the removal from the donor organism treated with adenosine, preferably for a period of time from about 30 to 20 minutes to about 10 to 5 minutes prior to the removal of the donor organ.

10. Use according to claim 1, whereby the condition is trauma.

11. Use according to claim 10, whereby the trauma results from the handling of an organ or part thereof.

12. Use according to claim 1, whereby the condition is ischemia.

13. Use according to claim 12, whereby the adenosine is administered for about 10 minutes to the patient about 20 to 10 minutes prior to ischemia.

14. Use according to claim 12 or 13, whereby the adenosine is administered to the patient for about 70 to 90 minutes after ischemia beginning about 1 to 5 minutes after removal of the ischemia causing blockade.

15. Use according to claim 1, whereby the condition is hypoxia.

16. Use according to any of claims 10 to 15, whereby the condition is arising from a surgery.

17. Use according to claim 16, whereby the condition is part of a medical treatment scheme.

18. Use according to claim 17, whereby the condition is an accidental event during such medical treatment scheme.

19. Use according to claims 17, whereby the condition is an intentional event during such medical treatment scheme.

20. Use according to claim 19, whereby the medical treatment scheme is related to the prevention and/or treatment of cerebral ischemia, trauma, hypoxia, acute Cor pulmonale, chronic Cor pulmonale intracerebral aneurysms.

21. Use according to claim 17, whereby the condition is part of a medical treatment scheme in which reanimation or the use of a heart-lung machine is involved.

22. Use of adenosine for the manufacture of a medicament for the treatment of multi-organ failure.

23. Method for systemic administration of adenosine to a patient comprising the steps of
(a) determining the hemodynamic situation of the patient;
(b) administering to the patient an amount of adenosine;
(c) determining the hemodynamic situation of the patient;
(d) comparing the hemodynamic situation of the patient with an undesired hemodynamic situation;
(e) repeating steps (b) to (d) in case the hemodynamic situation of the patient is different from the undesired hemodynamic situation, whereby the amount of adenosine administered is higher than the amount of adenosine administered in step (b)

24. The method according to claim 23, wherein the undesired hemodynamic situation is selected from the group comprising bradycardia, arrhythmia, and arterial hypotension.

25. The method according to claim 23 or 24, wherein upon realization or detection of the undesired situation for a period of time of at least 30 seconds, the amount of adenosine is administered which led to the undesired hemodynamic situation.

26. The method according to claim 25, wherein upon another occurrence of an or of the undesired hemodynamic situation, the amount of adenosine administered is adjusted to the one which does not trigger the undesired hemodynamic situation.

27. The method according to claim 26, wherein after about at least 30 seconds upon adjusting the amount of adenosine which does not trigger the undesired hemodynamic situation, the amount of adenosine administered is increased to the amount which triggers the undesired hemodynamic situation.
